# FASCICULE DE BREVET EUROPEEN

(11) **EP 3 591 031 B1**
(45) Date de publication et mention de la délivrance du brevet: **24.03.2021**
(21) Numéro de dépôt: 19179657.2
(22) Date de dépôt: 12.06.2019
(51) Int. Cl.: C12M 1/107, C12M 1/00

(54) **COUVERTURE AVEC COLLECTEUR D'EAU POUR CUVE DE METHANISATION**
ABDECKMODUL MIT WASSERSAMMLUNG FÜR BIOGASTANK
WATER COLLECTCING COVER FOR METHANATION TANK

(30) Priorité: 28.06.2018 FR 1855870
(43) Date de publication de la demande: 08.01.2020
(73) Titulaire: ADG - Ateliers Des Graves, 31270 Villeneuve Tolosane (FR)
(72) Inventeur: OF, Julien, 31270 VILLENEUVE TOLOSANE (FR); MORISAN, Richard, 31270 VILLENEUVE TOLOSANE (FR)
(74) Mandataire: Argyma

(56) Documents cités:
- EP-A1- 2 175 015
- WO-A2-2014/155368

## Description

### DOMAINE TECHNIQUE ET OBJET DE L'INVENTION

La présente invention concerne la méthanisation et plus particulièrement un module de couverture pour méthaniseur. Le module de couverture selon l'invention permet notamment le guidage et la collecte des eaux de pluie afin d'éviter la formation de poches d'eaux sur la couverture du module.

### ETAT DE LA TECHNIQUE

La méthanisation des matières organiques est un processus naturel anaérobie connu qui permet de transformer des matières organiques, notamment des matières agricoles, en biogaz. La méthanisation s'effectue par dégradation biologique des matières organiques par un consortium microbien comportant des bactéries. Il est ainsi notamment connu de transformer en biogaz la fraction fermentescible des effluents d'élevage produisant du lisier, tel que par exemple du lisier bovin, palmipède ou porcin, en disposant ces effluents dans une fosse à lisier aménagée dans le sol.

Afin de rendre la fosse au moins en partie étanche et améliorer l'efficacité de la méthanisation, il est connu de la recouvrir d'une bâche souple. Une telle solution ne permet toutefois pas de capter le biogaz issu de la transformation de la fraction fermentescible des effluents d'élevage. Aussi, lorsque l'on souhaite collecter le biogaz issu de la transformation de la fraction fermentescible de matières organiques, il est nécessaire de déposer les effluents dans une cuve de méthanisation, également appelée méthaniseur.

Un méthaniseur comprend de manière connue une cuve appelée digesteur, dans lequel on place les matières organiques, et un dispositif de collecte de biogaz. Dans un premier type de méthaniseur, la cuve est de forme cylindrique, fermée à ses extrémités et, par exemple, réalisée en métal. Les matières organiques sont acheminées à l'intérieur de la cuve par un conduit relié à la base de la cuve et le dispositif de collecte de biogaz se présente sous la forme d'un tuyau d'évacuation relié à la partie haute de la cuve. Dans un deuxième type de méthaniseur, la cuve est enfouie dans le sol, les matières organiques sont acheminées à l'intérieur de la cuve par un conduit et le dispositif de collecte de biogaz se présente sous la forme d'un module de couverture monté au-dessus de la cuve.

Ainsi, dans une solution décrite dans le document EP0827681B1, le module de couverture comprend une bâche souple montée sur une armature de support à laquelle est relié un tuyau d'évacuation du biogaz. L'armature de support de la bâche comporte une pluralité de poteaux métalliques fixés directement sur la cuve. Dans une autre solution, décrite dans la demande de brevet WO9828402A1, le module de couverture comprend un poteau central supportant une couverture tendue de sorte à former une former une structure pyramidale permettant de guider le biogaz vers le poteau où il est collecté via une pluralité d'orifice et de tuyaux WO2014155368 divulgue un méthanisateur comprenant une cuve et une couverture souple avec une zone de collecte des eaux de pluie et une structure de support.

Ces types de modules de couverture s'avèrent complexes et coûteux à fabriquer et à installer, ce qui présente des inconvénients importants. De plus, dans ces solutions, l'espace intérieur de la cuve est fixe de sorte que l'efficacité de la méthanisation varie avec le volume de matières organiques contenues dans la cuve. Notamment, lorsqu'il y a peu de matières organiques dans la cuve, le volume d'air stagnant au-dessus des effluents est tel que la méthanisation est lente et peu efficace, ce qui présente un inconvénient important. En outre, dans de telles solutions, les eaux de pluie ne sont pas collectées par le module de couverture et ne peuvent donc pas être réutilisées, notamment pour réaliser l'épandage nécessaire sur les matières organiques. Enfin, la conversion d'une fosse à lisier, réalisée en béton, en méthaniseur par la pose d'un module de couverture peut générer des contraintes mécaniques sur la fosse qui n'ont pas été prévues lors de sa conception et entrainer de facto des fissures dans le béton, ce qui présente un inconvénient majeur.

Afin de remédier au moins en partie à ces inconvénients, il est connu d'utiliser une bâche souple qui se déplace avec le volume de matières organiques. La couverture, réalisée en un matériau plastique souple, prend la forme d'un dôme surélevé dans sa zone centrale dont le volume intérieur permet de collecter le biogaz, les bords de la couverture reposant contre les parois de la cuve. Lorsqu'il pleut, les eaux de pluie s'accumulent dans la partie basse périphérique de la couverture. Ce faisant, les eaux de pluie peuvent former des poches dont le poids peut déformer le matériau souple de la zone périphérique et éloigner ainsi le bord de la couverture des parois de la cuve de sorte que l'atmosphère dans la cuve ne soit plus anaérobie, ce qui réduit significativement l'efficacité de la méthanisation. Il est donc nécessaire d'évacuer les eaux de pluie accumulées à la surface de la zone périphérique de la couverture.

Dans ce but, il est connu, d'utiliser une pompe qui évacue les eaux de pluie accumulées à la surface de la zone périphérique de la couverture. Or, d'un épisode pluvieux à un autre, les eaux de pluie peuvent s'accumuler à différents endroits de la zone périphérique de la couverture, non nécessairement sur toute la surface de ladite zone périphérique. Dans un tel cas, lorsque le ou les endroits de la zone périphérique de la couverture où se sont accumulées les eaux de pluie ne sont pas à portée de la buse d'aspiration de la pompe, elles ne peuvent pas être évacuées, ce qui peut éloigner le bord de la couverture des parois de la cuve et entrainer ainsi les inconvénients précités.

Il est donc nécessaire de déplacer la pompe de relevage à chaque endroit où se sont accumulées les eaux de pluie afin de les aspirer. Une telle méthode peut s'avérer particulièrement fastidieuse et chronophage et nécessite en outre la présence d'un ou plusieurs opérateurs pour déplacer la pompe de relevage sur chaque zone d'accumulation des eaux de pluie, ce qui peut s'avérer particulièrement coûteux et présente donc des inconvénients importants.

L'invention vise à résoudre au moins en partie ces inconvénients en proposant une solution de module de couverture qui soit à la fois fiable, efficace, robuste, rapide à installer et qui permette une évacuation efficace, rapide et aisée des eaux de pluie.

### PRESENTATION GENERALE DE L'INVENTION

A cet effet, l'invention a tout d'abord pour objet un module de couverture pour méthaniseur, ledit méthaniseur comprenant une cuve adaptée pour recevoir des matières organiques et comprenant un fond et au moins une paroi verticale délimitant une ouverture, ledit module de couverture comprenant :
- une couverture, réalisée en un matériau souple, configurée pour s'étendre dans ladite ouverture, ladite couverture comprenant une zone centrale et une zone périphérique et présentant une face externe, destinée à recevoir des eaux de pluie, et une face interne, destinée à s'étendre au-dessus des matières organiques,
- une structure de support reliée à la couverture au niveau de ladite zone périphérique et configurée à la fois pour maintenir ladite couverture au-dessus des matières organiques et pour se déplacer verticalement le long de la paroi de la cuve avec les matières organiques, le module de couverture étant remarquable en ce que la structure de support est configurée pour permettre le guidage, par la zone périphérique, des eaux de pluie reçues par la face externe de la couverture vers au moins un point de collecte permettant d'évacuer les eaux de pluie de ladite face externe.

Le module de couverture selon l'invention permet donc avantageusement d'acheminer les eaux de pluie vers un ou plusieurs points de collecte pour les évacuer de la face externe et éviter ainsi la formation de poches d'eaux de pluie sur ladite face externe. En particulier, la structure de support permet de rigidifier la zone périphérique afin d'éviter qu'elle ne se déforme sous le poids des eaux de pluie tout en guidant les eaux de pluie vers le ou les points de collecte. La zone périphérique, ainsi structurée par la structure de support, permet l'écoulement de l'eau sans obstacles, évitant ainsi la formation de poches. La rigidité de la zone périphérique permet de bien séparer lisier et eau de pluie. La structure de support permet de plaquer la bâche contre la ou les parois de la cuve afin d'éviter que la bâche ne monte trop haut en son centre. Le dôme formé au centre par le gonflage de la bâche draine les eaux de pluie vers la périphérie de la couverture qui les guident alors vers l'au moins un point de collecte. Le gonflage est ainsi équilibré de sorte que le module de couverture reste flottant et bien à plat au niveau de sa zone périphérique. En outre, la structure du module de couverture, notamment la structure de support et la couverture, permettent de créer une atmosphère anaérobie très rapidement au démarrage de la méthanisation, ce qui permet une croissance rapide des bactéries et donc de la production de biogaz tout en limitant les risques de formation d'atmosphère explosive, connue sous le nom d'ATEX (Atmosphère Explosive) dont le taux de dioxygène est supérieur à 6% et le taux de méthane est compris entre 5 et 15 % du total des gaz.

De préférence, la structure de support s'étend sur une largeur de la zone périphérique supérieure ou égale à 30 cm afin de rigidifier efficacement la couverture et guider ainsi efficacement les eaux de pluie vers le ou les points de collecte.

Avantageusement, la zone périphérique s'étend selon un plan sensiblement parallèle à l'horizontale terrestre (i.e. avec un angle de + ou - 3° par rapport à l'horizontale terrestre). Alternativement, la zone périphérique peut être inclinée par portion, par exemple d'un angle supérieur ou égal à + ou - 3°, de manière à guider rapidement et efficacement les eaux de pluie vers le ou les points de collecte.

Selon un aspect de l'invention, la structure de support comprend une armature extérieure reliée au bord de la couverture, et une armature intérieure, reliée d'une part à ladite armature extérieure et d'autre part à la couverture au niveau de la zone périphérique de sorte à rigidifier la surface de la zone périphérique comprise entre l'armature extérieure et l'armature intérieure.

Dans une forme de réalisation, l'armature extérieure comprend une pluralité d'éléments tubulaires reliés deux à deux par des éléments de liaison de manière à former un bord périphérique de flottaison du module de couverture.

Avantageusement, le bord du module de couverture est de forme polygonale ou de forme circulaire.

De préférence, les éléments tubulaires sont creux de manière à alléger la structure de support et rendre aisé le déplacement du module de couverture le long de la ou des parois de la cuve.

Dans une forme de réalisation, chaque élément de liaison comprend deux portions tubulaires, recevant chacune l'extrémité d'un élément tubulaire, et une plaque de support s'étendant depuis la partie inférieure des portions tubulaires en direction de la zone centrale de la couverture.

Avantageusement, la plaque de support s'étend sensiblement horizontalement. Par les termes « sensiblement horizontalement », on entend avec moins de 5° d'inclinaison par rapport à l'horizontale terrestre.

Avantageusement encore, la plaque de support est reliée à la couverture afin de rigidifier davantage la zone périphérique de la couverture.

Selon une caractéristique de l'invention, la couverture comprend une pluralité de passants et l'armature intérieure de la structure de support comprend au moins un câble reliant la structure de support, de préférence au niveau des plaques de support des éléments de liaison, à travers lesdits passants afin de rigidifier la zone périphérique et éviter la formation de poches d'eaux tout en guidant efficacement les eaux de pluie vers l'au moins un point de collecte.

Dans une forme de réalisation, deux éléments de liaison adjacents sont reliés par un même câble. Dans une forme de réalisation, le nombre de câbles est égal aux nombre d'éléments tubulaires. En variante, un unique câble peut relier tous les éléments de liaison entre eux.

Dans une forme de réalisation du module de couverture, le ou au moins l'un des points de collecte est un réservoir de collecte des eaux de pluie s'étendant depuis la face interne de la zone périphérique et débouchant sur la face externe de la zone périphérique.

De préférence, l'au moins un réservoir de collecte s'étend verticalement vers le bas depuis la face interne de la zone périphérique de la couverture.

Selon une caractéristique de l'invention, l'au moins un réservoir de collecte est formé dans la couverture, c'est-à-dire que l'au moins un réservoir de collecte est issu de matière de la couverture.

Avantageusement, le module de couverture comprend au moins une pompe de relevage montée au moins en partie, de préférence entièrement, dans le ou au moins l'un des réservoirs de collecte afin d'évacuer les eaux de pluie stockées dans ledit réservoir de collecte, par exemple via un tuyau d'évacuation. De préférence, chaque réservoir de collecte comprend une pompe de relevage.

Dans une forme de réalisation, le module de couverture comprend une pluralité de réservoirs de collecte disposés le long de la zone périphérique de la couverture afin de collecter efficacement et rapidement les eaux de pluie.

De manière avantageuse, les réservoirs de collecte sont équirépartis autour de la zone périphérique de la couverture de sorte que le volume des eaux de pluie soit sensiblement le même dans chaque réservoir de collecte, ce qui permet d'éviter un déséquilibre des masses et une déformation de la couverture.

Dans une forme de réalisation, chaque réservoir de collecte est disposé au droit d'une plaque de support d'un élément de liaison de l'armature extérieure de la structure de support, une ouverture étant formée dans ladite plaque de support afin de permettre aux eaux de pluie de pénétrer dans le réservoir de collecte.

En variante ou en complément, le ou au moins l'un des points de collecte est une buse d'aspiration d'une pompe de relevage. La pompe de relevage peut par exemple être montée à l'extérieur de la cuve de sorte à aspirer les eaux de pluie hors du module de couverture via un tuyau d'évacuation.

De préférence, le module de couverture comprend au moins un capteur de tension permettant de contrôler le gonflage de la couverture sur toute la surface de ladite couverture afin de s'assurer que le dôme formé par la couverture est régulier lors de l'utilisation du module de couverture.

L'invention concerne également un méthaniseur comprenant une cuve et un module de couverture, tel que présenté précédemment, ladite cuve étant adaptée pour recevoir des matières organiques et comprenant un fond et au moins une paroi délimitant une ouverture, ledit module de couverture étant disposé dans la cuve de sorte à s'étendre dans ladite ouverture.

Avantageusement, la structure de support n'est pas liée à la paroi de la cuve afin de simplifier le module de couverture et la cuve ainsi que l'installation du module de couverture dans la cuve.

### PRESENTATION DES FIGURES

L'invention sera mieux comprise à la lecture de la description qui va suivre, donnée uniquement à titre d'exemple, et se référant aux dessins annexés donnés à titre d'exemples non limitatifs, dans lesquels des références identiques sont données à des objets semblables et sur lesquels :
- la figure 1 est une vue en perspective d'une forme de réalisation d'un méthaniseur selon l'invention,
- la figure 2 est une vue en coupe du méthaniseur de la figure 1,
- la figure 3 est une vue partielle en perspective du module de couverture du méthaniseur de la figure 1,
- la figure 4 est une autre vue partielle en perspective du module de couverture du méthaniseur de la figure 1,
- la figure 5 est une vue partielle en perspective du module de couverture du méthaniseur de la figure 1,
- la figure 6 est une vue partielle en coupe et en perspective du module de couverture du méthaniseur de la figure 1.

Il faut noter que les figures exposent l'invention de manière détaillée pour mettre en oeuvre l'invention, lesdites figures pouvant bien entendu servir à mieux définir l'invention le cas échéant.

### DESCRIPTION DETAILLEE DE L'INVENTION

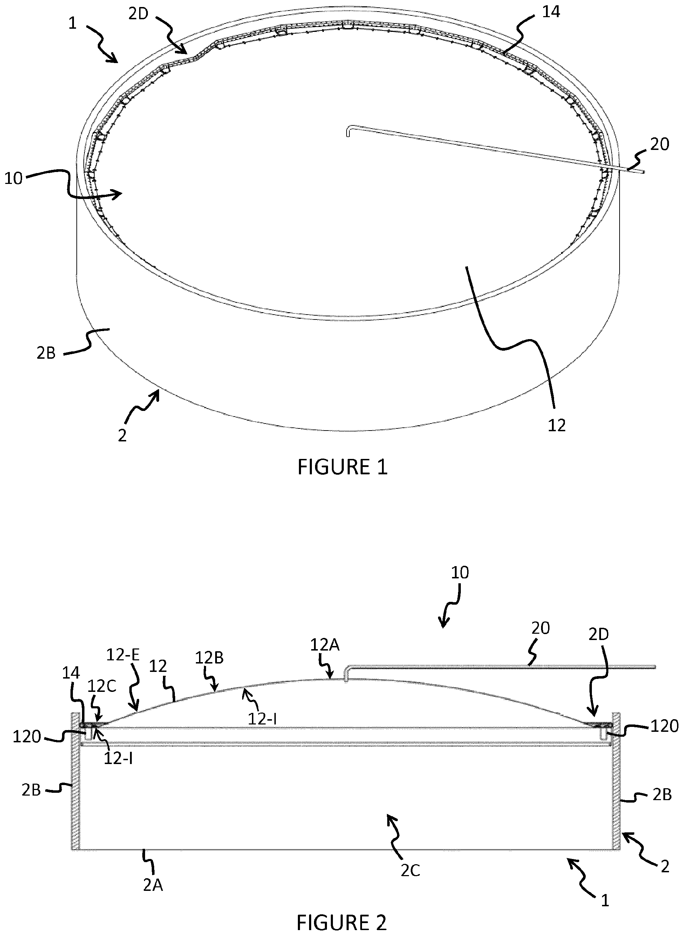

On a représenté sur les figures 1 et 2 une forme de réalisation d'un méthaniseur 1 selon l'invention. Tout d'abord, en référence à la figure 1, le méthaniseur 1 comprend une cuve 2, dans laquelle sont placées des matières organiques (non visibles), et un module de couverture 10. Le méthaniseur 1 permet la transformation de la fraction fermentescible des matières organiques contenues dans la cuve 2, le module de couverture 10 permettant de capter et évacuer le biogaz issu de ladite transformation.

En référence à la figure 2, la cuve 2, par exemple réalisée en béton, comprend dans cet exemple un fond 2A et une paroi 2B verticale cylindrique de section circulaire. Le fond 2A et la paroi 2B délimitent un espace interne 2C, adapté pour recevoir des matières organiques, débouchant sur une ouverture 2D dans laquelle s'étend le module de couverture 10 de manière à l'obturer.

Le module de couverture 10 comprend une couverture 12 et une structure de support 14 sur laquelle est fixée ladite couverture 12.

La couverture 12 est réalisée en un matériau souple (prenant par exemple la forme d'une toile), de préférence en un matériau plastique, apte à prendre une forme de dôme pendant la collecte du biogaz issu de la transformation de la fraction fermentescible des matières organiques. L'évacuation du biogaz collecté est réalisée via un tuyau d'évacuation 20 vers l'extérieur du méthaniseur 1, par exemple vers un module de stockage de biogaz (non représenté).

La couverture 12 comprend une zone centrale 12A, une zone médiane 12B circulaire et une zone périphérique 12C, toutes trois circulaires. La couverture 12 est reliée à la structure de support 14 au niveau de la zone périphérique 12C. La face supérieure de la couverture 12 constitue la face externe 12-E de la couverture 12, c'est-à-dire la face tournée vers le ciel afin de recevoir les eaux de pluie lors d'un épisode pluvieux. La face inférieure de la couverture 12 constitue la face interne 12-I de la couverture 12 et est tournée vers l'intérieur de la cuve 2 tout en recouvrant les matières organiques.

Dans la forme de réalisation illustrée, le module de couverture 10 comprend une pluralité de points de collecte des eaux de pluie se présentant sous la forme de réservoirs de collecte 120 formés dans la zone périphérique 12C de la couverture 12. De préférence, les réservoirs de collecte 120 sont équirépartis autour de la zone périphérique 12C de la couverture 12, c'est-à-dire que les réservoirs de collecte 120 sont régulièrement espacés tout autour de la zone périphérique 12C. Bien entendu, en variante, les réservoirs de collecte 120 pourraient être répartis différemment, notamment de manière aléatoire, le long de la zone périphérique 12C. De même, dans une autre forme de réalisation, la couverture 12 pourrait ne comprendre qu'un unique réservoir de collecte 120. Enfin, on notera que les réservoirs de collecte 120 peuvent présenter différentes dimensions selon le niveau de collecte et de stockage souhaité, sans que cela ne soit limitatif de la portée de la présente invention.

De préférence, chaque réservoir de collecte 120 est formé dans le matériau souple constituant la couverture 12 en étant issu de matière dudit matériau. Chaque réservoir de collecte 120 s'étend vers le bas sous la couverture 12 depuis la face interne 12-I de la zone périphérique 12C de la couverture 12 et débouche dans sa partie supérieure au niveau de la face externe 12-E de la couverture 12 de manière à collecter les eaux de pluie par gravité.

Une unité de flottaison centrale optionnelle (non représentée), disposée dans la cuve 2 sur les matières organiques, peut être prévue afin de supporter la couverture 12 au niveau de sa zone centrale 12A. Lorsque le module de couverture 10 est dépourvu d'unité de flottaison centrale, la surélévation de la zone centrale 12A de la couverture 12 peut être conférée par une armature supportant le matériau souple constituant la couverture 12 ou bien directement par la pression générée par le biogaz stocké sous la couverture 12.

La structure de support 14 est reliée à la couverture 12 au niveau de sa zone périphérique 12C de sorte à maintenir le module de couverture 10 au-dessus des matières organiques et à permettre son déplacement vertical le long de la paroi 2B de la cuve 2, notamment pour accompagner les augmentations ou réductions de volume des matières organiques.

La structure de support 14 est configurée pour permettre le guidage, par la zone périphérique 12C, des eaux de pluie reçues par la face externe 12-E de la couverture 12 vers le ou les points de collecte 120 afin d'évacuer les eaux de pluie de ladite face externe 12-E.

La structure de support 14 comprend une armature extérieure, reliée au bord de la couverture 12 et une armature intérieure, reliée d'une part à ladite armature extérieure et d'autre part à la couverture 12 au niveau de la zone périphérique 12C de sorte à rigidifier la portion de la zone périphérique 12C comprise entre l'armature extérieure et l'armature intérieure.

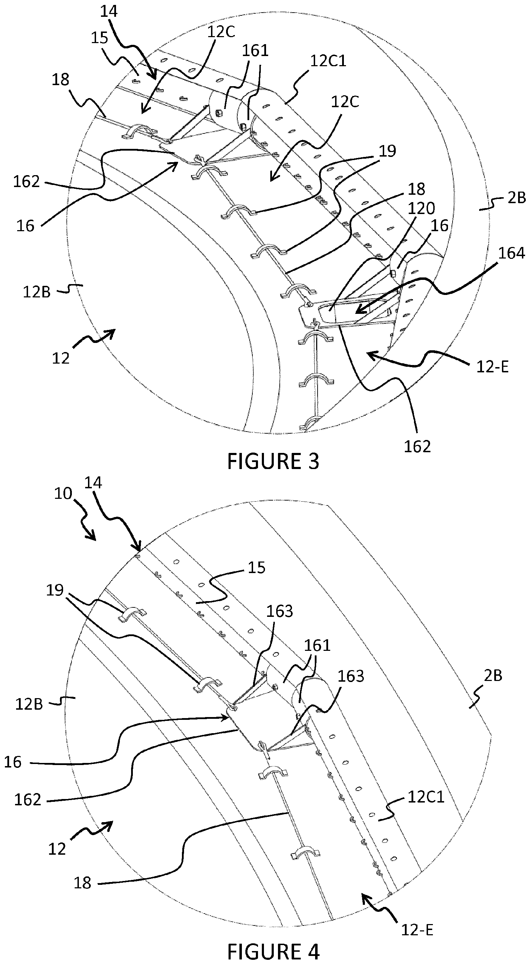

Dans l'exemple non limitatif illustré sur les figures 3 à 5, l'armature extérieure est de forme polygonale et comprend une pluralité d'éléments tubulaires 15 reliés entre eux deux à deux par des éléments de liaison 16, chaque élément tubulaire 15 constituant un côté du polygone.

Chaque élément de liaison 16 est monobloc et métallique et comprend deux portions tubulaires 161, une plaque de support 162 et deux tiges de liaison 163 permettant de renforcer l'élément de liaison 16.

Chaque portion tubulaire 161 est adaptée pour recevoir l'extrémité d'un élément tubulaire 15 de manière à former l'assemblage de la structure de support 14. Dans cet exemple, les deux portions tubulaires 161 ne sont pas coaxiales mais leurs axes longitudinaux forment un angle inférieur à 180° permettant de conférer sa forme polygonale à la structure de support 14.

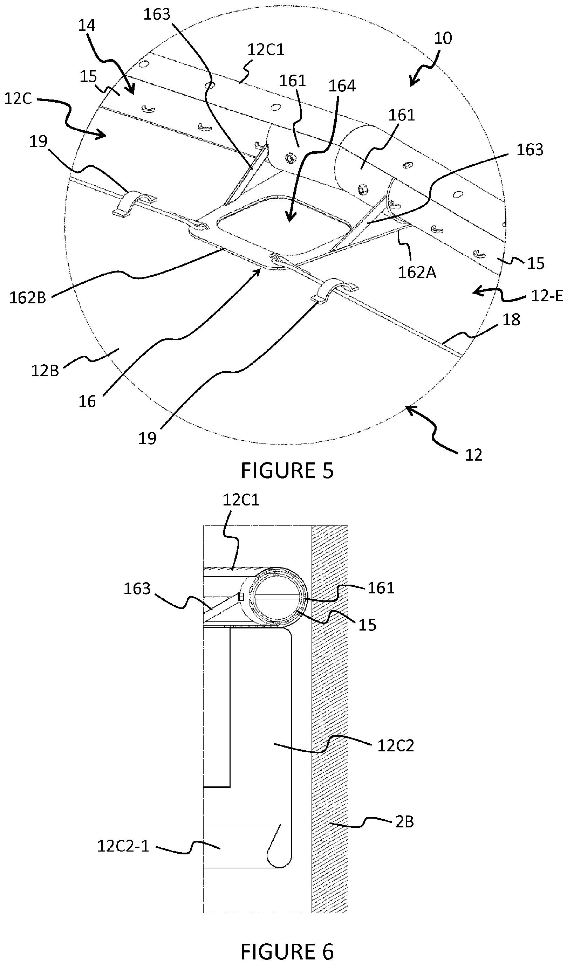

La plaque de support 162 s'étend depuis la surface inférieure des deux portions tubulaires 161 selon un plan sensiblement horizontal et est reliée aux deux portions tubulaires 161, au niveau de leur zone médiane, par deux tiges de liaison 163 plates afin de rigidifier l'élément de liaison 16. Plus précisément, comme illustré sur la figure 5, chaque plaque de support 162 comprend une extrémité de liaison 162A reliée aux deux portions tubulaires 161 et une extrémité libre 162B s'étendant en direction de la zone centrale 12A de la couverture 12, les deux tiges de liaison 163 étant reliées à la plaque de support 162 au niveau de son extrémité libre 162B.

L'armature intérieure comprend une pluralité de câbles 18 reliant les plaques de support 162 de l'armature extérieure deux à deux. Plus précisément, deux plaques de support 162 de deux éléments de liaison 16 adjacents sont reliées par un câble 18 dont chaque extrémité est fixée au niveau de l'extrémité libre 162B de la plaque de support 162 qu'elle relie. Des passants 19 permettent de maintenir la couverture 12 fixée aux câbles 18 afin de rigidifier la structure de support 14 et notamment de maintenir la zone périphérique 12C sensiblement horizontale.

Les éléments de liaison 16, les câbles 18 et les passants 19 permettent ainsi de maintenir le matériau souple de la zone périphérique 12C de la couverture 12 tendu et sensiblement horizontal de sorte que ladite la zone périphérique 12C guide les eaux de pluie vers les réservoirs de collecte 120 et évite ainsi la formation de poches d'eaux de pluie sur la face externe 12-E de la couverture 12. Autrement dit, les éléments de liaison 16, notamment les plaques de support 162, les câbles 18 et les passants 19 constituent des organes de maintien qui permettent à la fois d'empêcher la déformation de la zone périphérique 12C par les eaux de pluie, notamment en la maintenant sensiblement horizontal, et de guider lesdites eaux de pluie vers les réservoirs de collecte 120 pour éviter que les eaux de pluie ne s'accumulent et ne déforment la zone périphérique 12C.

Dans la forme de réalisation décrite, non limitative, chaque réservoir de collecte 120 est disposé au droit d'une plaque de support 162 d'un élément de liaison 16. Afin de collecter les eaux de pluie, une ouverture 164 est formée dans la plaque de support 162 placée au-dessus de chaque réservoir de collecte 120 afin de permettre le passage des eaux de pluie à travers ladite ouverture 164 jusque dans ledit réservoir de collecte 120.

De préférence, la structure de support 14 empêche la déformation de la zone périphérique 12C au moins sur une largeur supérieure ou égale à 30 cm. Autrement dit, la longueur des plaques de support 162 est supérieure ou égale à 30 cm.

En référence à la figure 6, la couverture 12 comprend une première portion 12C1, issue de matière de la couverture 12, enroulée en partie autour des éléments tubulaires 15 pour fixer la couverture 12 à la structure de support 14, et une deuxième portion 12C2, également issue de matière de la couverture 12, s'étendant vers le bas, depuis les éléments tubulaires 15, le long de la paroi 2B de la cuve 2 et servant à lester la structure de support 14 afin que le bord du module de couverture 10 reste plaqué le plus possible contre la paroi 2B de la cuve 2. A cette fin, dans cet exemple, la deuxième portion 12C2 comprend un bord inférieur 12C2-1 recourbé autour d'une barre de lestage (non visible), par exemple réalisée en métal.

De préférence, chaque réservoir de collecte 120 comprend une pompe de relevage, (non représentée), par exemple de type vide-cave, permettant de pomper les eaux de pluie stockées dans les réservoirs de collecte 120. Ces pompes de relevage permettent d'évacuer les eaux de pluie stockées dans les réservoirs de collecte 120 vers l'extérieur du module de couverture 10, notamment afin d'éviter que les réservoirs de collecte 120 débordent et déforment la zone périphérique 12C.

## Revendications

1. Module de couverture (10) pour méthaniseur (1), ledit méthaniseur comprenant une cuve (2) adaptée pour recevoir des matières organiques et comprenant un fond (1A) et au moins une paroi (1B) verticale délimitant une ouverture (1C), ledit module de couverture (10) comprenant :
- une couverture (12), réalisée en un matériau souple, configurée pour s'étendre dans ladite ouverture (1C), ladite couverture (12) comprenant une zone centrale (12A) et une zone périphérique (12C) et présentant une face externe (12-E), destinée à recevoir des eaux de pluie, et une face interne (12-I), destinée à s'étendre au-dessus des matières organiques,
- une structure de support (14) reliée à la couverture (12) au niveau de ladite zone périphérique (12C) et configurée à la fois pour maintenir ladite couverture (12) au-dessus des matières organiques et pour se déplacer verticalement le long de la paroi (1B) de la cuve (1) avec les matières organiques,
le module de couverture (10) étant **caractérisé en ce que** la structure de support (14) est configurée pour permettre le guidage, par la zone périphérique (12C), des eaux de pluie reçues par la face externe (12-E) de la couverture (12) vers au moins un point de collecte (120) permettant d'évacuer les eaux de pluie de ladite face externe (12-E).

2. Module de couverture (10) selon la revendication 1, dans lequel la structure de support (14) s'étend sur une largeur de la zone périphérique (12C) supérieure ou égale à 30 cm.

3. Module de couverture (10) selon l'une quelconque des revendications précédentes, dans lequel la structure de support (14) comprend une armature extérieure, reliée au bord de la couverture (12) et une armature intérieure, reliée d'une part à ladite armature extérieure et d'autre part à la couverture (12) au niveau de la zone périphérique (12C) de sorte à rigidifier la surface de la zone périphérique (12C) comprise entre l'armature extérieure et l'armature intérieure.

4. Module de couverture (10) selon la revendication précédente, dans lequel l'armature extérieure comprend une pluralité d'éléments tubulaires (15) reliés deux à deux par des éléments de liaison (16).

5. Module de couverture (10) selon la revendication précédente, dans lequel chaque élément de liaison (16) comprend deux portions tubulaires (161), recevant chacune l'extrémité d'un élément tubulaire (15), et une plaque de support (162) s'étendant depuis la partie inférieure des portions tubulaires (161) en direction de la zone centrale (12A) de la couverture (12).

6. Module de couverture (10) selon la revendication précédente, dans lequel la couverture (12) comprend une pluralité de passants (19) et l'armature intérieure de la structure de support (14) comprend au moins un câble (18) reliant la structure de support, de préférence au niveau des plaques de support (162) des éléments de liaison (16), à travers lesdits passants (19).

7. Module de couverture (10) selon l'une quelconque des revendications précédentes, dans lequel l'au moins un point de collecte est un réservoir de collecte (120) des eaux de pluie s'étendant depuis la face interne (12-I) de la zone périphérique (12C) et débouchant sur la face externe (12-E) de la zone périphérique (12C).

8. Module de couverture (10) selon la revendication précédente, dans lequel l'au moins un réservoir de collecte (120) s'étend verticalement vers le bas depuis la face interne de la zone périphérique (12C) de la couverture (12).

9. Module de couverture (10) selon l'une quelconque des revendications 7 et 8, dans lequel l'au moins un réservoir de collecte (120) est issu de matière de la couverture (12).

10. Module de couverture (10) selon l'une quelconque des revendications 7 à 9, ledit module de couverture (10) comprenant au moins une pompe de relevage montée au moins en partie dans l'au moins un réservoir de collecte (120) afin d'évacuer les eaux de pluie stockées dans ledit réservoir de collecte (120).

11. Module de couverture (10) selon l'une quelconque des revendications 7 à 10, ledit module de couverture (10) comprenant une pluralité de réservoirs de collecte (120), lesdits réservoirs de collecte (120) étant équirépartis autour de la zone périphérique (12C) de la couverture (12).

12. Module de couverture (10) selon l'une quelconque des revendications 7 à 11, dans lequel l'au moins un réservoir de collecte (120) est disposé au droit d'une plaque de support (162) d'un élément de liaison (16) de l'armature extérieure de la structure de support (14), une ouverture (164) étant formée dans ladite plaque de support (162) afin de permettre aux eaux de pluie de pénétrer dans le réservoir de collecte (120).

13. Module de couverture (10) selon l'une des revendications précédentes, dans lequel l'au moins un point de collecte est une buse d'aspiration d'une pompe de relevage.

14. Module de couverture (10) selon l'une quelconque des revendications précédentes, comprenant au moins un capteur de tension permettant de contrôler le gonflage de la couverture (12) sur toute la surface de ladite couverture (12).

15. Méthaniseur (1) comprenant une cuve (2) et un module de couverture (10), selon l'une quelconque des revendications précédentes, ladite cuve (2) étant adaptée pour recevoir des matières organiques et comprenant un fond (2A) et au moins une paroi (2B) délimitant une ouverture (2D), ledit module de couverture (10) étant disposé dans la cuve (2) de sorte à s'étendre dans ladite ouverture (2D).

## Patentansprüche

1. Abdeckmodul (10) für Biogaserzeuger (1), wobei der Biogaserzeuger einen Tank (2) umfasst, der zur Aufnahme der organischen Materialien geeignet ist und einen Boden (1A) und mindestens eine vertikale Wand (1B) umfasst, die eine Öffnung (1C) begrenzt, wobei das Abdeckmodul (10) umfasst:
- eine Abdeckung (12), die aus einem elastischen Material hergestellt ist, die ausgelegt ist, um sich in der Öffnung (1C) zu erstrecken, wobei die Abdeckung (12) eine zentrale Zone (12A) und eine periphere Zone (12C) umfasst und eine Außenfläche (12-E) aufweist, die zur Aufnahme des Regenwassers bestimmt ist, und eine Innenfläche (12-I), die bestimmt ist, sich oberhalb der organischen Materialien zu erstrecken,
- eine tragende Struktur (14), die mit der Abdeckung (12) im Bereich der peripheren Zone (12C) verbunden und ausgelegt ist, um sowohl die Abdeckung (12) oberhalb der organischen Materialien zu halten als auch um sich vertikal entlang der Wand (1B) des Tanks (1) mit den organischen Materialien zu verlagern,
wobei das Abdeckmodul (10) **dadurch gekennzeichnet ist, dass** die tragende Struktur (14) ausgelegt ist, um die Führung, durch die periphere Zone (12C), des von der Außenfläche (12-E) der Abdeckung (12) aufgenommenen Regenwassers zu mindestens einem Sammelpunkt (120) zu erlauben, um die Ableitung des Regenwassers von der Außenfläche (12-E) zu erlauben.

2. Abdeckmodul (10) nach Anspruch 1, wobei sich die tragende Struktur (14) über eine Breite der peripheren Zone (12C) erstreckt, die größer oder gleich 30 cm ist.

3. Abdeckmodul (10) nach einem der vorangehenden Ansprüche, wobei die tragende Struktur (14) eine äußere Armierung umfasst, die mit dem Rand der Abdeckung (12) verbunden ist, und eine innere Armierung, die zum einen mit der äußeren Armierung und zum anderen mit der Abdeckung (12) im Bereich der peripheren Zone (12C) derart verbunden ist, dass die Oberfläche der peripheren Zone (12C) zwischen der äußeren Armierung und der inneren Armierung versteift wird.

4. Abdeckmodul (10) nach vorangehendem Anspruch, wobei die äußere Armierung eine Vielzahl rohrförmiger Elemente (15) umfasst, die durch Verbindungselemente (16) paarweise verbunden sind.

5. Abdeckmodul (10) nach vorangehendem Anspruch, wobei jedes Verbindungselement (16) zwei rohrförmige Abschnitte (161) umfasst, die jeweils das Ende eines rohrförmigen Elements (15) aufnehmen, und eine tragende Platte (162), die sich von dem unteren Teil der rohrförmigen Abschnitte (161) in Richtung der zentralen Zone (12A) der Abdeckung (12) erstreckt.

6. Abdeckmodul (10) nach vorangehendem Anspruch, wobei die Abdeckung (12) eine Vielzahl von Durchgängen (19) umfasst und die innere Armierung der tragenden Struktur (14) mindestens ein Kabel (18) umfasst, das die tragende Struktur, vorzugsweise im Bereich der tragenden Platten (162) der Verbindungselemente (16), durch die Durchgänge (19) verbindet.

7. Abdeckmodul (10) nach einem der vorangehenden Ansprüche, wobei der mindestens eine Sammelpunkt ein Sammelbehälter (120) des Regenwassers ist, der sich ab der Innenfläche (12-I) der peripheren Zone (12C) erstreckt und auf der Außenfläche (12-E) der peripheren Zone (12C) ausmündet.

8. Abdeckmodul (10) nach vorangehendem Anspruch, wobei sich der mindestens eine Sammelbehälter (120) ab der Innenfläche der peripheren Zone (12C) der Abdeckung (12) vertikal nach unten erstreckt.

9. Abdeckmodul (10) nach einem der Ansprüche 7 und 8, wobei der mindestens eine Sammelbehälter (120) aus dem Material der Abdeckung (12) hervorgegangen ist.

10. Abdeckmodul (10) nach einem der Ansprüche 7 bis 9, wobei das Abdeckmodul (10) mindestens eine Hebepumpe umfasst, die mindestens teilweise in dem mindestens einen Sammelbehälter (120) angebracht ist, um das in dem Sammelbehälter (120) gelagerte Regenwasser abzuleiten.

11. Abdeckmodul (10) nach einem der Ansprüche 7 bis 10, wobei das Abdeckmodul (10) eine Vielzahl von Sammelbehältern (120) umfasst, wobei die Sammelbehälter (120) um die periphere Zone (12C) der Abdeckung (12) gleichmäßig verteilt sind.

12. Abdeckmodul (10) nach einem der Ansprüche 7 bis 11, wobei der mindestens eine Sammelbehälter (120) senkrecht zu einer tragenden Platte (162) eines Verbindungselements (16) der äußeren Armierung der tragenden Struktur (14) angeordnet ist, wobei in der tragenden Platte (162) eine Öffnung (164) ausgebildet ist, um dem Regenwasser zu erlauben, in den Sammelbehälter (120) einzudringen.

13. Abdeckmodul (10) nach einem der vorangehenden Ansprüche, wobei der mindestens eine Sammelpunkt eine Ansaugdüse einer Hebepumpe ist.

14. Abdeckmodul (10) nach einem der vorangehenden Ansprüche, umfassend mindestens einen Spannungssensor, der erlaubt, die Aufblasung der Abdeckung (12) auf der gesamten Oberfläche der Abdeckung (12) zu überprüfen.

15. Biogaserzeuger (1), umfassend einen Tank (2) und ein Abdeckmodul (10) nach einem der vorangehenden Ansprüche, wobei der Tank (2) zur Aufnahme organischer Materialien geeignet ist und einen Boden (2A) und mindestens eine Wand (2B) umfasst, die eine Öffnung (2D) begrenzt, wobei das Abdeckmodul (10) derart im Tank (2) angeordnet ist, dass es sich in der Öffnung (2D) erstreckt.

## Claims

1. A cover module (10) for a methanizer (1), said methanizer comprising a vessel (2) adapted to receive organic matters and comprising a bottom (1A) and at least one vertical wall (1B) delimiting an aperture (1C), said cover module (10) comprising:
- a cover (12), made of a flexible material, configured to extend into said aperture (1C), said cover (12) comprising a central zone (12A) and a peripheral zone (12C) and having an outer face (12-E), for receiving rainwater, and an inner face (12-1), for extending above organic matters,
- a support structure (14) connected to the cover (12) at said peripheral zone (12C) and configured both to hold said cover (12) above organic matters and to move vertically along the wall (1B) of the vessel (1) with organic matters,
the cover module (10) being **characterised in that** the support structure (14) is configured to enable, through the peripheral zone (12C), rainwater received by the outer face (12-E) of the cover (12) to be guided towards at least one collecting point (120) enabling rainwater to be discharged from said outer face (12-E).

2. The cover module (10) according to claim 1, wherein the support structure (14) extends over a width of the peripheral zone (12C) greater than or equal to 30cm.

3. The cover module (10) according to any of the previous claims, wherein the support structure (14) comprises an external framework, connected to the edge of the cover (12) and an internal framework, connected on the one hand to said external framework and on the other hand to the cover (12) at the peripheral zone (12C) so as to rigidify the surface of the peripheral zone (12C) comprised between the external framework and internal framework.

4. The cover module (10) according to the previous claim, wherein the external framework comprises a plurality of tubular elements (15) connected two by two by connecting elements (16).

5. The cover module (10) according to the previous claim, wherein each connecting element (16) comprises two tubular portions (161), each receiving the end of a tubular element (15), and a support plate (162) extending from the lower part of the tubular portions (161) towards the central zone (12A) of the cover (12).

6. The cover module (10) according to the previous claim, wherein the cover (12) comprises a plurality of basic frames (19) and the internal framework of the support structure (14) comprises at least one cable (18) connecting the support structure, preferably at the support plates (162) of the connecting elements (16), through said basic frames (19).

7. The cover module (10) according to any of the previous claims, wherein the at least one collecting point is a rainwater collecting tank (120) extending from the inner face (12-1) of the peripheral zone (12C) and opening into the outer face (12-E) of the peripheral zone (12C).

8. The cover module (10) according to the previous claim, wherein the at least one collecting tank (120) extends vertically downwards from the inner face of the peripheral zone (12C) of the cover (12).

9. The cover module (10) according to any of claims 7 to 8, wherein the at least one collecting tank (120) is of the same material as the cover (12).

10. The cover module (10) according to any of claims 7 to 9, said cover module (10) comprising at least one lift pump at least partly mounted into the at least one collecting tank (120) in order to discharge rainwater stored in said collecting tank (120).

11. The cover module (10) according to any of claims 7 to 10, said cover module (10) comprising a plurality of collecting tanks (120), said collecting tanks (120) being equally distributed about the peripheral zone (12C) of the cover (12).

12. The cover module (10) according to any of claims 7 to 11, wherein the at least one collecting tank (120) is disposed at right angles with a support plate (162) of a connecting element (16) of the external framework of the support structure (14), an aperture (164) being formed into said support plate (162) in order to enable rainwater to penetrate the collecting tank (120).

13. The cover module (10) according to one of the previous claims, wherein the at least one collecting point is a suction nozzle of a lift pump.

14. The cover module (10) according to any of the previous claims, comprising at least one tension sensor for controlling swelling of the cover (12) over the whole surface of said cover (12).

15. A methanizer (1) comprising a vessel (2) and a cover module (10), according to any of the previous claims, said vessel (2) being suitable to receive organic matters and comprising a bottom (2A) and at least one wall (2B) delimiting an aperture (2D), said cover module (10) being disposed into the vessel (2) so as to extend into said aperture (2D).
